(19) Europäisches Patentamt
European Patent Office
Office européen des brevets

(11) **EP 4 494 684 A1**

(12) **EUROPEAN PATENT APPLICATION**

(43) Date of publication:
**22.01.2025 Bulletin 2025/04**

(21) Application number: **23186128.7**

(22) Date of filing: **18.07.2023**

(51) International Patent Classification (IPC):
*A61M 16/08* (2006.01)    *A61M 16/00* (2006.01)
*G01F 1/36* (2006.01)    *G01F 1/40* (2006.01)
*G01F 1/42* (2006.01)    *G01F 1/44* (2006.01)
*G01F 1/684* (2006.01)    *G01F 1/688* (2006.01)
*G01F 5/00* (2006.01)    *G01F 15/00* (2006.01)
*G01F 15/14* (2006.01)

(52) Cooperative Patent Classification (CPC):
**A61M 16/0816; G01F 1/363; G01F 1/40;
G01F 1/42; G01F 1/44; G01F 1/6845; G01F 1/6888;
G01F 5/00; G01F 5/005; G01F 15/006; G01F 15/14;**
A61M 16/0084; A61M 16/161; A61M 2016/0027;
A61M 2016/0039;                          (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Sensirion AG
8712 Stäfa (CH)**

(72) Inventors:
• **BRUEHWILER, Markus
8608 Bubikon (CH)**

• **HAASE, Janine
8820 Waedenswil (CH)**
• **HORNUNG, Mark
8712 Staefa (CH)**
• **ZIPKES, Christoph
8632 Tann (CH)**

(74) Representative: **Detken, Andreas
Isler & Pedrazzini AG
Giesshübelstrasse 45
Postfach 1772
8027 Zürich (CH)**

(54) **DEVICE AND METHOD FOR MONITORING A FLOW OF A FLUID**

(57)    A monitoring device for monitoring a flow of a fluid like a breathing gas comprises a main flow path (10) for the fluid between an inlet (11) and an outlet (12). A flow sensor (15) is associated with the main flow path. A flow restrictor (14) is arranged in the main flow path. A secondary flow path (20) for the fluid branches off from the main flow path upstream of the flow restrictor and merges with the main flow path downstream of the flow restrictor. The secondary flow path has higher flow resistance than the main flow path. At least one fluid property sensor (24; 25) for determining at least one property of the fluid in the secondary flow path (20) is associated with the secondary flow path (20). Also disclosed is a ventilation system that comprises the monitoring device and a patient interface (30).

**FIG. 1**

(52) Cooperative Patent Classification (CPC): (Cont.)
A61M 2205/3334; A61M 2205/3344;
A61M 2205/3606; A61M 2230/432;
A61M 2230/435

**Description**

TECHNICAL FIELD

The present invention relates to a device and a method for monitoring a flow of a fluid. The device may be integrated into a resuscitation device, in particular, into a bag-valve-mask device.

PRIOR ART

Resuscitation devices are essential tools used in emergency medical situations to assist in restoring circulation and breathing for individuals experiencing cardiac arrest or respiratory distress. These devices are designed to deliver oxygen to support the vital functions of the body until further medical intervention is available. They thus play a crucial role in improving survival rates during cardiac arrest or respiratory emergencies.

A commonly used resuscitation device is the bag-valve-mask (BVM) device. This handheld device comprises a self-inflating bag connected to a breathing mask, allowing a rescuer to manually provide positive pressure ventilation to a non-breathing or inadequately breathing patient.

Although standard bag-valve-mask resuscitators have the potential to deliver efficient life support, ensuring their proper utilization is far from assured. Even highly skilled practitioners may encounter difficulties in operating the device correctly. One prevalent error in using bag-valve-mask resuscitators is the inadequate sealing of the mask against the patient's face, leading to air leaks. Additionally, other frequently observed mistakes that compromise effective resuscitation include obstructed airways and improper ventilation frequency and volume.

In order to increase the likelihood that the air is correctly delivered to the patient, it has been proposed in the prior art to determine the air flow rate and pressure of the air flowing through a bag-valve-mask resuscitator. In this way, medical personnel can receive real-time feedback about resuscitation quality and respiration of the patient. Examples are provided in WO2020118138A1 and WO2014078840A1.

However, measurements obtained with such known devices can be unreliable. In particular, the pressure readings can strongly deviate from the actual pressure in the mouthpiece. Similar problems also exist in mechanical ventilation devices as commonly used in hospitals.

There is therefore a need for systems and methods to further improve the monitoring of a fluid flow.

SUMMARY OF THE INVENTION

It is an object of the present invention to provide a device for monitoring a fluid flow that ensures more reliable and accurate readings of the monitored parameters than hitherto possible.

This object is achieved by a monitoring device as defined in claim 1. Further embodiments are laid down in the dependent claims.

A device for monitoring a flow of a fluid is provided. The device comprises:

> a main flow path for the fluid between a main path inlet and a main path outlet;
> a flow sensor coupled to the main flow path for measuring a fluid flow rate in the main flow path;
> a flow restrictor arranged in the main flow path to cause a pressure difference across the flow restrictor when the fluid flows along the main flow path;
> a secondary flow path for the fluid, the secondary flow path being in fluidic communication with the main flow path, the secondary flow path bypassing the flow restrictor, the secondary flow path having higher flow resistance than the main flow path; and
> at least one fluid property sensor coupled to the secondary flow path for determining at least one property of the fluid in the secondary flow path.

By measuring the flow rate in the main flow path, high sensitivity is obtained especially at low fluid flow rates. This results in an improved signal-to-noise ratio for the flow measurement, reducing the need for time consuming averaging of the signal. Therefore, higher sampling rates and faster response times are possible with such a design. Measuring the flow rate in the main flow path also results in a more linear raw sensor signal at low flows. This reduces unwanted side-effects when averaging and filtering in noisy systems, where inaccuracies are introduced through the non-linear characteristic. It further improves the accuracy of the flow measurement because the linearization function will have less degrees of freedom and will thus better reproduce the raw signal characteristic.

Measuring the at least one fluid property in the secondary flow path opens the way for optimizing physical and environmental conditions that contribute to the performance of the at least one fluid property sensor. These conditions may be optimized independently from the main flow path, which can be independently tuned for optimal flow measurement performance.

The fluid may be a liquid or a gas. The fluid may in particular be a breathing gas, more particularly air, possibly enriched with oxygen, more specifically, breathing air to be supplied to a patient for ventilation or exhaled air received from a patient. In some embodiments, the monitoring device may be part of a resuscitation device, e.g., a bag-valve-mask device, or of a mechanical ventilator. To this end, the outlet of the monitoring device may be connected to a patient interface, e.g., to a breathing mask. However, the monitoring device may also be useful in other applications where it is desired to simultaneously determine a fluid flow rate and at least one property of the fluid with high reliability and high accuracy.

The monitoring device comprises a flow restrictor in the main flow path to create a pressure gradient along the secondary flow path when the fluid is flowing along the main flow path. This pressure gradient in turn causes a flow of the fluid through the secondary flow path. More specifically, the secondary flow path may have a secondary path inlet and a secondary path outlet. The secondary flow path may branch off from the main flow path at the secondary path inlet upstream of the flow restrictor and merge with the main flow path at the secondary path outlet downstream of the flow restrictor.

The flow restrictor may be formed, e.g., by a plate having one or more orifices, thus locally reducing the cross-sectional area available for the flow. In other embodiments, the flow restrictor may be formed by a constriction of the primary flow channel. Many other designs that act as flow restrictors to cause a pressure gradient along the secondary flow path are possible. By a careful design of the flow restrictor, the flow conditions not only in the primary flow channel, but also in the secondary flow channel can be influenced to aid in meeting the above design objectives.

The secondary flow path has higher flow resistance than the main flow path. This causes the flow rate in the secondary flow path to be small compared to the flow rate in the main flow path. In particular, it may be advantageous if the secondary flow path has higher flow resistance than the main flow path by a factor of not less than 100 and preferably of not more than 10'000. The term "flow resistance" is to be understood as designating the derivative $(\partial(\Delta p))/\partial Q$ of the pressure difference $\Delta p$ between the upstream and downstream ends (or between the inlet and the outlet) of a flow path with respect to the volume flow rate $Q$ along the flow path. For a laminar flow and an incompressible fluid, the pressure difference will generally be proportional to the volume flow rate, and the flow resistance then is the factor of proportionality. If the flow is not strictly laminar and/or if the fluid is not incompressible, the pressure difference may depend in a non-linear manner on the volume flow rate, or the volume flow rate may depend in a non-linear manner on the pressure difference. In such cases, the flow resistance will itself depend on the volume flow rate or pressure difference. The flow resistance may therefore be considered at a specified volume flow rate or at a specified pressure difference. In the present case, it may be useful to consider the flow resistance of the main flow path at a volume flow rate of 1 liter per second through the main flow path, and to consider the flow resistance of the secondary flow path at a pressure difference that is caused between the inlet and outlet of the secondary flow path due to said volume flow rate through the main flow path. With the presently proposed design, a typical value of this pressure difference may be about 1 to 3 mbar. The flow resistance further generally depends on the viscosity of the fluid and may thus depend on properties of the fluid that affect its viscosity, e.g., on its temperature and pressure. The flow resistance may therefore be considered at a specified temperature, e.g., at a standard temperature of 20 °C, and at a specified pressure, e.g., at a standard pressure of 1013.25 mbar at the outlet end of the flow path. If the monitoring device is intended to be used with air, the flow resistances of the main and secondary flow paths are preferably determined for dry air at standard conditions (20 °C and 1013.25 mbar).

The flow resistance of a flow path can readily be tailored by adjusting the length and cross-sectional profile of the flow channel that defines the flow path, and/or by providing a suitable flow restrictor in the flow path. By causing the secondary flow path to have higher flow resistance than the main flow path, preferably by a factor of at least 100, it is ensured that the volume flow rate through the secondary flow path will be significantly lower than the flow rate through the main flow path. Thereby disturbance of the flow in the main flow path can be minimized, and the flow in the secondary flow path can be made sufficiently low to allow the gas in the secondary flow path to acquire a uniform temperature. On the other hand, the flow resistance of the secondary flow path should not be too high to ensure that the secondary flow path is permanently flooded with fresh fluid, thus ensuring rapid response of the at least one fluid property sensor in the secondary flow path. A good practical upper limit for the ratio of the flow resistances of the secondary and main flow paths may be 10'000.

In some embodiments, the main flow path includes a flow-metering flow path that bypasses the flow restrictor, and the flow sensor may be arranged in the flow-metering flow path. By providing a quasi-bypass for the flow sensor, the flow conditions for the flow sensor can be further optimized, and the flow sensor can be placed in a location that is readily accessible for mounting. The position along the main flow path where the flow-metering flow path branches off from the main flow path is preferably downstream of the position where the secondary flow path branches off from the main flow path, and the position where the flow-metering flow path opens out into the main flow path again is preferably upstream of the position where the secondary flow path opens out into the main flow path. The flow resistance of the secondary flow path is preferably higher than the flow resistance of the flow-metering flow path, preferably at least by a factor of 10, to cause the flow rate through the flow-metering flow path to be higher than the flow rate through the secondary flow path. A higher flow rate through the flow-metering flow path facilitates more accurate flow rate measurements.

In some embodiments, the monitoring device may comprise a heat exchanger arranged to be in thermal contact with the fluid in the secondary flow path. The heat exchanger may be configured, in particular, to reduce or minimize temperature gradients along the secondary flow path in a region where the at least one fluid property sensor is arranged. Temperature gradients across the at least one fluid property sensor can be detrimental to the accuracy of the fluid property sensor. The heat exchanger may comprise a material having high thermal conductivity that extends along at least a portion of the secondary flow path between an upstream portion of the secondary flow path upstream of the at least one fluid property sensor and a downstream portion of the secondary flow path downstream of the at least one fluid property sensor, the material being in thermal contact with the fluid in the secondary flow path to facilitate heat exchange between the fluid in the upstream portion and the fluid in the downstream portion. A material may be considered to have "high thermal conductivity" if its thermal conductivity is at least 10 W/(m · K). The material may be, for example, a metal or a compound material comprising a metal, e.g., a multi-layer material having a metal core layer coated on one or both sides with a polymeric surface layer. The heat exchanger preferably has a heat transfer coefficient between the upstream and downstream portions of the secondary flow channel that is higher than a heat transfer coefficient between these portions due to forced convection of the fluid through the secondary flow channel. In particular, the heat exchanger may be configured to cause a heat transfer rate between the upstream and downstream portions of the secondary flow channel by heat conduction through the heat exchanger that is higher than the heat transfer rate due to forced convection of the fluid through the secondary flow channel when a temperature difference exists between the upstream and downstream portions.

In addition or in the alternative, the heat exchanger may be configured to facilitate heat exchange of the fluid in the secondary flow path with an environment outside of the monitoring device in order to thermalize the fluid in the secondary flow path with the environment, e.g., , causing the fluid in the secondary flow path to assume a similar temperature or essentially the same temperature as the environment. In this manner, the at least one physical property of the fluid in the secondary flow path can be determined under conditions that are closer to environmental conditions, and as such significantly more stable, than the conditions in the main flow path. This is useful, in particular, when the fluid composition is determined, for example, when carbon dioxide content in air is determined using a microthermal sensor that measures thermal conductivity, as the result of a composition measurement will be more accurate in a stable environment, particularly, when there are no fast temperature changes. The heat exchanger may comprise a material having high thermal conductivity that extends between the secondary flow path and the environment to facilitate heat exchange between the fluid in the secondary flow path and the environment.

The heat exchanger may comprises (or may be formed by) a carrier on which the flow sensor and/or the at least one fluid property sensor are mounted. As noted above, the carrier may comprise a material having high thermal conductivity that extends along at least a portion of the secondary flow path between an upstream portion and a downstream portion of the secondary flow path to facilitate heat exchange between the fluid in the upstream portion and the fluid in the downstream portion. In particular, the carrier may be a circuit board comprising at least one metal layer, the metal layer facilitating heat transfer along a length of the circuit board. Preferably, the metal layer defines at least one uninterrupted heat conduction path between the upstream and downstream portions of the secondary flow channel. In addition or in the alternative, the carrier may comprise a material having high thermal conductivity that extends between the secondary flow path and the environment to facilitate heat exchange between the fluid in the secondary flow path and the environment. If the carrier is a circuit board, metal may extend, for example, through through-holes (vias) in the circuit board.

The carrier may form part of a housing of the monitoring device, the carrier delimiting the secondary flow path. In addition or in the alternative, the heat exchanger may comprise (or may be formed by) any other portion of the housing of the monitoring device that delimits the secondary flow path. Specifically, the housing portion may be formed of a material having high thermal conductivity along a length of the housing and/or between an inside surface of the housing portion.

Other important design parameters that affect heat exchange with the environment are the cross-section of the secondary flow path and the geometry of its surface. For a given cross-sectional area, the surface area should preferably be large. In order to ensure a large surface area, it is preferred if a cross section of the secondary flow path has an aspect ratio (ratio of width and height measured in a plane that is perpendicular to a principal direction of flow in the secondary flow path) of at least 5:1 in the region where the fluid in the secondary flow path is in contact with the heat exchanger.

In some embodiments, the device may comprise a heater in thermal contact with the secondary flow path for selectively heating the fluid in the secondary flow path. The heater may be operable to prevent condensation of gases into liquids in the secondary flow path or to remove condensate that may have already formed. The heater may be in thermal contact with the heat exchanger to rapidly heat the fluid in the secondary flow path. If the heat exchanger comprises a carrier on which the flow sensor and/or the at least one fluid property sensor are mounted, the heater preferably is mounted on the carrier.

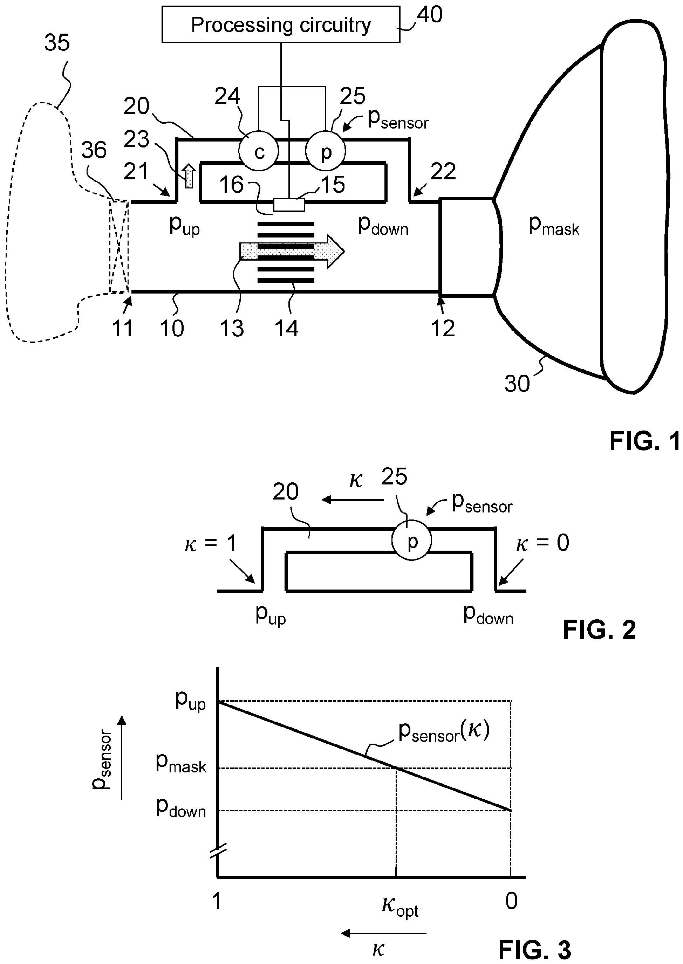

In some embodiments, the at least one fluid property sensor includes a pressure sensor for measuring a fluid pressure of the fluid in the secondary flow path. By associating the pressure sensor with the secondary flow path rather than the main flow path, the pressure readings may more accurately reflect the pressure of the fluid at an actual point of interest. Specifically, the pressure sensor may be arranged to measure the fluid pressure in the secondary flow path at a position that corresponds to a fraction $\kappa_{opt} = -(1 + \zeta_B)/\zeta_A$ of a total length of the secondary flow path, measured from the downstream end of the secondary flow path (or the secondary path outlet), negative sign of the fraction meaning that the position is upstream of the downstream end, where $\zeta_A$ is a pressure loss coefficient at the flow restrictor and $\zeta_B$ is a pressure loss coefficient at the main path outlet. In practice, the fraction $\kappa_{opt}$ will often be between 0.2 and 0.8. Further considerations in this respect will be explained below with reference to Fig. 1.

The monitoring device may be configured to determine a standardized flow rate based on the measured fluid flow rate in the main flow path and on the measured fluid pressure in the secondary flow path. For example, if the flow sensor is a mass flow sensor, the monitoring device may be configured to determine a volume flow rate at standard conditions from the measured mass flow rate, using the measured pressure and a measured, known or assumed temperature. In particular, the monitoring device may comprise a temperature sensor for determining the temperature of the fluid in the main flow path.

In some embodiments, the at least one fluid property sensor includes a fluid composition sensor for determining a composition of the fluid in the secondary flow path. The fluid composition sensor may be provided instead of or in addition to the pressure sensor. For example, in a ventilation or resuscitation device, additional information about the dynamically changing gas composition during a breathing cycle can be obtained in this manner. This information may be used for providing additional feedback to medical personnel performing the ventilation. For example, in a resuscitation system, information from the fluid composition sensor may be provided to a resuscitation-assistance algorithm that is executed by processing circuitry of the monitoring device. The resuscitation-assistance algorithm may output diagnostic information and/or instructions, taking into account the information of the fluid composition sensor. By coupling the fluid composition sensor to the secondary flow channel, the dynamically changing composition can be determined rapidly, accurately and under controlled conditions.

The monitoring device may be configured to carry out the steps of:

determining a flow direction in the primary flow channel based on signals from the flow sensor to discriminate between inhalation and exhalation events; and
determining output data that selectively indicate the at least one property of the fluid in the secondary flow path, in particular, its composition, during inhalation and/or exhalation events.

If the fluid is a breathing gas, its composition during both inhalation and exhalation can be monitored in this manner, e.g., its oxygen content during inhalation can be ascertained, and important information about vital functions of the patient can be obtained.

In some embodiments, the fluid composition sensor may be configured to determine a concentration of at least one of the following components of the fluid in the secondary flow path:

carbon dioxide ($CO_2$);
molecular oxygen ($O_2$);
argon (Ar);
nitric oxide (nitrogen monoxide, NO);
acetone; and
humidity.

The levels of molecular oxygen and carbon dioxide in exhaled breath provide important information about respiratory function, the metabolism, and overall health status of the patient. Argon is important as a measure of the purity of oxygen used for ventilation. Nitric oxide and acetone are important additional diagnostic markers in exhaled breath. Humidity information can be used, for example, to correct output from other fluid property sensors for cross-sensitivities to humidity changes.

In some embodiments, the monitoring device may be configured to carry out a compensation of the measured fluid flow rate for variations of the measured fluid composition. For example, if the flow sensor is a mass flow sensor, the monitoring device may be configured to determine a volume flow rate at standard conditions from the mass flow rate, using the measured information about the fluid composition, the measured pressure and a measured, known or assumed temperature.

A broad range of fluid composition sensors may be employed. For example the fluid composition sensor may be a sensor of one of the following types:

thermal sensors;
semiconductor sensors;
optical sensors; and
electrochemical sensors.

Thermal sensors are preferred because they are robust and small, can be manufactured at low cost with integrated circuitry, have short reaction time. A "thermal sensor" is a sensor that detects fluid composition by measuring one or more heat transfer properties of the fluid, in particular, thermal conductivity, specific heat and/or thermal diffusivity. Thermal sensors usually comprise at least one heater or cooler for creating a thermal stimulus and at least one temperature sensor for determining a thermal response of the sensor to the thermal stimulus. The thermal response depends on the heat transfer properties of the fluid that is in contact with the thermal sensor. Based on the thermal response, the concentration of a target component of the fluid can be calculated. Such a sensor may be employed, for example, for determining the carbon dioxide content in exhaled breath.

A "semiconductor sensor" is a sensor that detects fluid composition by a change in electrical resistance due to adsorption of a constituent of the fluid or due to a chemical reaction that takes place when the fluid comes in direct contact with the sensor. This change in resistance is used to calculate the concentration of a target constituent of the fluid. Semiconductor sensors are commonly used to detect humidity, hydrogen, oxygen, ethanol vapor, and harmful gases such as carbon monoxide.

An "optical sensor" determines the composition of a fluid by measuring absorption of light in the fluid. Optical sensors are commonly used to measure, e.g., oxygen, carbon dioxide and nitric oxide in air.

"Electrochemical sensors" are sensors that measure the concentration of a target component in a fluid by oxidizing or reducing the target component at an electrode and measuring the resulting current.

In some embodiments, the flow sensor and the at least one fluid property sensor (e.g., pressure sensor and fluid composition sensor) are mounted on a common carrier, e.g., on a common circuit board. This allows cost-efficient assembly of the device and enables communication between the sensors and other circuitry with relatively little effort. As explained above, the carrier may in addition act as a heat exchanger or may form part thereof.

The device may comprise a housing in which the main and secondary flow paths are arranged or formed. The carrier may form part of the housing. In particular, the housing may define the main flow path. To this end, the housing may comprise a tubular portion that defines the main flow path. The tubular portion may have a circular cross section. The housing may further define a receptacle that is shaped to receive the carrier with the flow sensor and the at least one fluid property sensor mounted thereon. Openings may be present between the main flow path and the receptacle, the openings forming the secondary path inlet and the secondary path outlet such that the secondary flow path passes through the receptacle. The housing may further define a first cavity for receiving the flow sensor, the first cavity having an opening towards the main flow path. In this manner, fluid flowing along the main flow path is able to get in contact with the flow sensor when the flow sensor is received in the first cavity. The first cavity may advantageously be configured in such a manner that it is able to receive the flow sensor in a fluid-tight manner when the carrier with the flow sensor and the at least one fluid property sensor mounted thereon is received in the receptacle. The housing may further define at least one second cavity for receiving the at least one fluid property sensor when the carrier with the flow sensor and the at least one fluid property sensor mounted thereon is received in the receptacle, such that fluid in the secondary flow path can contact the at least one fluid property sensor. The first and second cavities may have a common orientation such that the flow sensor and the at least one fluid property sensor can be inserted into their associated cavities by moving the circuit board with the flow sensor and at least one fluid property sensor mounted thereon into the receptacle along an insertion direction.

The monitoring device may comprise processing circuitry for operating the flow sensor and the at least one fluid property sensor and for deriving output signals based on the outputs of these sensing devices. In particular, the processing circuitry may be configured to carry out the steps of:

for a plurality of time intervals, operating the flow sensor and the at least one fluid property sensor to obtain the fluid flow rate in the main flow path and the at least one property of the fluid in the secondary flow path;

determining a flow direction in the primary flow channel based on signals from the flow sensor to discriminate between inhalation and exhalation events;

determining separate output data for the inhalation and exhalation events of one or more of the breathing cycles based on the fluid flow rate and/or the at least one property of the fluid; and

optionally, outputting the output data.

As explained above, the monitoring device, in particular, its processing circuitry, may be configured to determine output data that selectively indicate the composition of the fluid in the secondary flow path during inhalation and/or exhalation events, and/or to correct the measured fluid flow rate for variations in fluid composition based on the measured fluid composition, and/or to generate feedback for personnel performing the ventilation, e.g., in the form of handling instructions.

The monitoring device may further comprise a display that is connected to the processing circuitry for displaying the output data. The display may be physically connected to the processing circuitry by a wired data connection, or it may be connected via a wireless data connection. The display may be mounted on or in the housing of the monitoring device, or it may be provided as a separate device.

In order to facilitate use of the monitoring device in a ventilation system, the main path inlet may be shaped as a connector for connection to a source of a breathing gas, and the main path outlet may be shaped as a connector for attachment to a patient interface.

The present invention further provides a ventilation system for ventilating a patient, in particular, a resuscitation system, comprising the monitoring device and a patient interface attached to the outlet of the monitoring device. The patient interface may in particular be a breathing mask, i.e., a mask that covers the mouth and possibly other parts of the face or head, designed to direct the fluid from the outlet of the primary flow channel to the patient's mouth. The ventilation system may in particular be a mobile ventilation system for use in resuscitation. The ventilation system may further comprise a manual breathing gas delivery device comprising a self-inflating bag.

If the at least one fluid property sensor includes a pressure sensor for measuring a fluid pressure in the secondary flow path, the pressure sensor may be arranged at a position where the fluid pressure in the secondary flow path substantially corresponds to a pressure of the fluid in the patient interface when the patient receives the fluid through the monitoring device and the attached patient interface. A pressure as determined by a pressure sensor is considered to "substantially correspond" to the pressure of the fluid in the patient interface if the deviation between these two pressure values at a flow rate of 1 liter per second is not more than 0.5 mbar, preferably not more than 0.2 mbar, more preferably not more than 0.1 mbar. As will be explained in more detail below in conjunction with Figure 1, the pressure measured by the pressure sensor and the pressure in the patient interface can be matched by an appropriate choice of position of the pressure sensor along the secondary flow path.

A method of operating a ventilation system as disclosed above may comprise carrying out the following steps during a plurality of breathing cycles, each breathing cycle comprising an inhalation event and an exhalation event:

operating the flow sensor and the at least one fluid property sensor to obtain the fluid flow rate in the main flow path and the at least one property of the fluid in the secondary flow path during inhalation events and exhalation events;

determining a flow direction in the primary flow channel based on signals from the flow sensor to discriminate between inhalation and exhalation events;

determining separate output data for the inhalation and exhalation events of one or more of the breathing cycles based on the fluid flow rate and/or the at least one property of the fluid; and

optionally, outputting the output data.

In the above description, the terms "upstream" and "downstream" are to be understood to be positions relative to a flow from an "inlet" end to an "outlet" end of a flow path. However, this shall not exclude that a flow occurs in the opposite direction, from the outlet end to the inlet end of the flow path. For example, if the monitoring device is included in a ventilation device, flow occurs from the inlet end to the outlet end of each of the main and secondary flow paths during an inhalation event, whereas flow occurs in the opposite direction during the exhalation event.

BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

Fig. 1    shows, in a schematic longitudinal section, a monitoring device according to a first embodiment together with a patient interface in the form of a breathing mask;

Fig. 2    shows a detail view of the monitoring device in Fig. 1 for illustrating the meaning of a variable $\kappa$;

Fig. 3    shows a diagram illustrating, in a schematic and simplified manner, a dependence of a measured pressure in the secondary flow path as a function of the variable $\kappa$;

Fig. 4    shows, in a schematic longitudinal section, a monitoring device according to a second embodiment;

Figs. 5-7  show, in a schematic cross-sectional view, various possibilities for arranging the main and secondary flow paths;

Fig. 8    shows, in a perspective view, a portion of a monitoring device according to an embodiment of the present disclosure;

Fig. 9    shows, in a partial perspective view, the portion of Fig. 8 from a different viewing direction;

Fig. 10   shows a printed circuit board carrying a flow sensor, a pressure sensor and a fluid composition sensor;

Fig. 11   shows metal-filled hole in a printed circuit board;

Fig. 12   shows an exemplary embodiment of a thermal flow sensor; and

Fig. 13   shows an exemplary embodiment of processing circuitry.

DESCRIPTION OF PREFERRED EMBODIMENTS

Setup of a monitoring device

Figure 1 illustrates a monitoring device according to a first embodiment. The monitoring device is part of a resuscitation device, e.g., a bag-valve-mask device.

The monitoring device comprises a main fluid channel defining a main flow path 10 and a secondary fluid channel defining a secondary flow path 20. The main flow path 10 extends from an inlet 11 to an outlet 12. A main flow 13 flows along the main flow path 10. In the example of Fig. 1, the flow direction of the main flow 13 is from the inlet 11 to the outlet 12; however, the flow direction can also be the opposite direction.

A flow restrictor 14 is arranged in the main flow path 10, dividing the main flow path 10 into an upstream portion and a downstream portion relative to the flow restrictor 14. The secondary flow path 20 branches off from the upstream portion of the main flow path 10 and opens out into the downstream portion of the main flow path 10.

The main flow 13 causes a pressure difference across the flow restrictor 14. Due to this pressure difference, the pressure $p_{up}$ at the upstream end of the secondary flow path is higher than the pressure $p_{down}$ at its downstream end. This pressure difference causes a secondary flow 23 along the secondary flow path 20. Typically, at a flow rate of 1 liter per second of the main flow 13, the pressure difference will be between 1 and 3 mbar.

The secondary flow path has higher flow resistance than the main flow path. The flow resistance of the secondary flow path can be tailored by appropriately choosing the dimensions of the secondary flow path, in particular, its length and cross-sectional profile. In the present example, the minimum cross-sectional area of the secondary flow path is significantly smaller than the minimum cross-sectional area of the primary flow path. Therefore, the secondary flow 23 has much smaller flow rate than the main flow 13.

A flow sensor 15 is associated with the main flow path 10 in such a manner that a portion of the main flow 13 sweeps over the flow sensor 15. The flow sensor 15 is arranged in a flow-metering flow path 16 that branches off from the main flow path upstream of the flow restrictor 14 and merges with the main flow path 10 downstream of the flow restrictor 14, thereby bypassing the flow restrictor 14. The flow-metering flow path 16 is shorter than the secondary flow path 20, branching off from the main flow path 10 downstream of the upstream end of the secondary flow path 20 and opening out into the main flow path 10 upstream of the downstream of the secondary flow path 20. Its flow resistance is lower than the flow resistance of the secondary flow path 20.

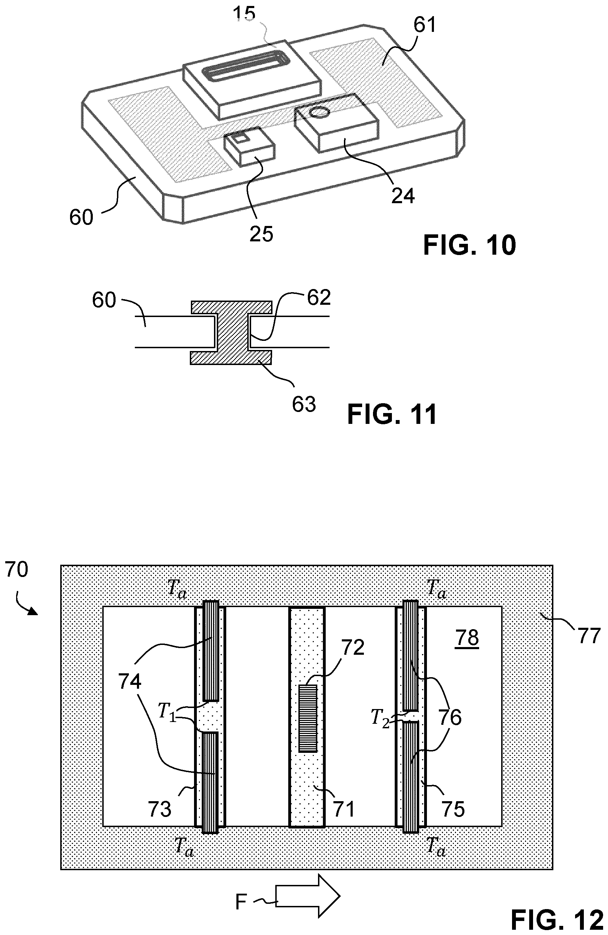

The flow sensor 15 may be a microthermal mass flow sensor, as it is known in the prior art. An example will be discussed below in conjunction with Fig. 12. However, other types of flow sensors may be used.

Two fluid property sensors are associated with the secondary flow path 20: a fluid composition sensor 24 for determining at least one parameter that is indicative of the composition of the fluid in the secondary flow path, and a pressure sensor 25 for determining the fluid pressure $p_{sensor}$ in the secondary flow path 20.

The fluid composition sensor 24 may be, for example, a concentration sensor for determining the concentration $c$ of some component of the fluid in the secondary flow path 20. In particular, the fluid composition sensor 24 may be a microthermal sensor for determining a heat transfer parameter, in particular, thermal conductivity of the fluid in the secondary flow path 20. As thermal conductivity correlates with the concentration of certain constituents of the fluid, a measurement of thermal conductivity allows conclusions about the composition of the fluid in the secondary flow path 20. For example, the thermal conductivity of carbon dioxide strongly differs from the thermal conductivity of dry air. By measuring thermal conductivity, conclusions can be drawn about the carbon dioxide concentration in air.

However, other types of fluid composition sensors may also be used, in particular, semiconductor sensors, optical sensors or electrochemical sensors. Several sensors of the same or different types may be combined, the sensors being sensitive to different gases. For example, carbon dioxide concentration may be determined with a microthermal sensor, oxygen concentration may be determined with a first semiconductor sensor, and humidity may be determined with a second semiconductor sensor. Optionally, trace gases like carbon monoxide, ethanol vapor and nitric oxide may be determined using further sensors. In this manner, the fluid can be characterized on a detailed level.

The pressure sensor 25 may be an absolute or differential pressure sensor. For example, the pressure sensor may be configured as a differential pressure sensor to determine a pressure difference between the secondary flow path 20 and the environment outside the monitoring device. This may be done by measuring a mass flow along a very narrow channel that connects the secondary flow path 20 with the environment. A microthermal sensor may be used for this purpose. In other embodiments, the pressure sensor 25 is an absolute or gauge pressure sensor as it is known from the prior art.

Processing circuitry 40 operates the flow sensor 15 and the fluid property sensors 24, 25 and derives output data from the measurement results.

The outlet 12 of the monitoring device is connected to a patient interface 30 in the form of a breathing mask 30. The inlet 11 of the monitoring device may be connected to a pressure source (illustrated only in a highly schematic manner as self-inflating bag 35) and three-way valve (illustrated only in a highly schematic manner as valve 36) allowing air to flow from the pressure source through the monitoring device to the patient interface for ventilation and exhaled air from the patient interface to escape to the environment after the exhaled air has passed the monitoring device. The pressure source may be a self-inflatable bag of a BVM device.

Flow impedance matching

The pressure sensor 15 is positioned in the secondary flow path in such a manner that the pressure $p_{sensor}$ measured by the pressure sensor 15 substantially corresponds to the static pressure $p_{mask}$ inside the patient interface 30.

This is done based on the following considerations:

a) The goal is to place the pressure sensor such that $p_{mask} - p_{sensor} \equiv 0$, i.e. the static pressure in the patient interface $p_{mask}$ should be equal to the static pressure $p_{sensor}$ at the pressure sensor 25 at all times. It is assumed that dynamic pressures in the secondary flow channel 20 and in the patient interface 30 are negligible and that the fluid's potential energy is the same in all places.

b) The pressure at the position of the pressure sensor 25 is $p_{sensor} = p_{down} - \kappa(p_{down} - p_{up}) = p_{down} - \kappa\zeta_A p_{dyn}$, where $\kappa$ is a position parameter which describes the position of the pressure sensor within the secondary flow path in a simple linear model and $\zeta_A$ is an empirical pressure loss coefficient of the flow restrictor 14. The pressure loss coefficient $\zeta_A$ is a factor of proportionality between the dynamic pressure $p_{dyn}$ in the main flow path 10 and the pressure drop across the flow restrictor 14. Its sign is negative.

The dynamic pressure $p_{dyn} = \frac{1}{2}\rho v^2$ is assumed to be the same everywhere in the main flow path 10 and the pressure drop over the flow restrictor thus is $\zeta_A p_{dyn} < 0$.

c) In the patient interface 30, dynamic pressure is reduced to zero. The static pressure in the patient interface thus is $p_{mask} = (1 + \zeta_B)p_{dyn} + p_{down}$, with $\zeta_B$ being an empirical pressure loss coefficient at the main path outlet 12 and $\zeta_B p_{dyn} < 0$ being the pressure drop at the main path outlet 12.

d) Therefore, $p_{mask} - p_{sensor} = (1 + \zeta_B + \kappa\zeta_A)p_{dyn}$ and $\kappa_{opt} = -(1 + \zeta_B)/\zeta_A$ for the desired condition where $p_{mask} - p_{sensor} \equiv 0$.

Therefore, to determine the optimal placement of the pressure sensor 25, the following steps are carried out:

> a) Determine the two pressure loss coefficients $\zeta_A$ and $\zeta_B$ by appropriate measurements.
> b) Calculate the optimal pressure sensor position parameter $\kappa_{opt} = -(1 + \zeta_B)/\zeta_A$.
> c) Place the pressure sensor at the corresponding position.

If $\kappa_{opt}$ is not between 0 and 1 (i.e., if $|\zeta_A| < 1 + \zeta_B$), the pressure loss coefficients $\zeta_A$ and/or $\zeta_B$ can be appropriately adjusted by changing the flow resistance at the flow restrictor 14 and/or by changing the flow resistance at the main path outlet 12.

The procedure is illustrated graphically in Figures 2 and 3.

Figure 2 visualizes the meaning of the position parameter $\kappa$. At the secondary path inlet 21, the value of the parameter $\kappa$ is 1. At the secondary path outlet 22, its value is zero. The value of $\kappa$ continuously changes from 1 to 0 along the length of the secondary flow path 20 from its inlet to its outlet. The parameter $\kappa$ thus describes a position along the secondary flow path.

Figure 3 shows the dependence of the measured pressure $p_{sensor}$ as a function of the position parameter $\kappa$. The optimum value $\kappa_{opt}$ is obtained when $p_{sensor}$ matches $p_{mask}$.

## Alternative embodiment: flow restrictor, heat exchanger and heater

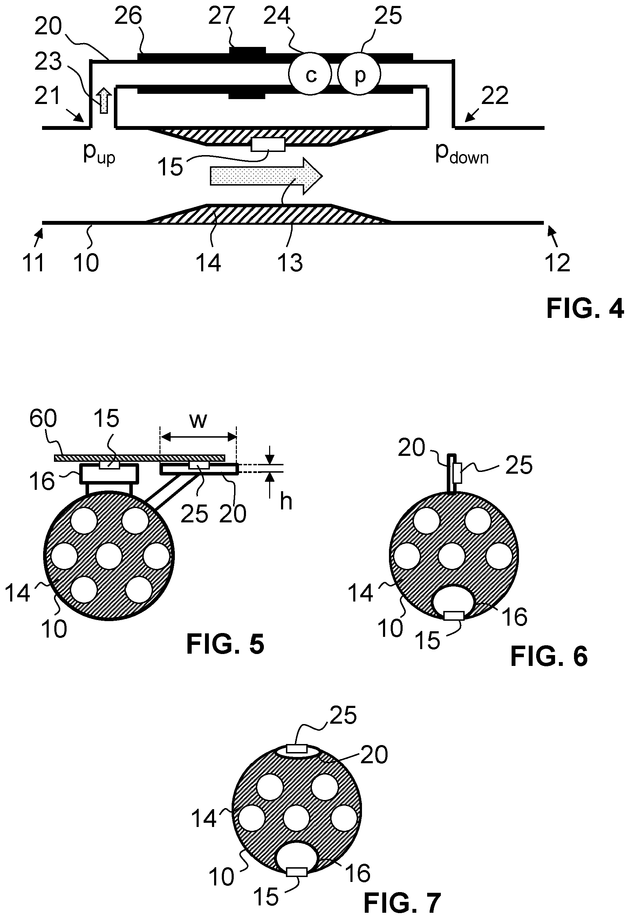

Figure 4 illustrates an alternative embodiment of a monitoring device. For simplicity, the patient interface 30 is not shown.

In contrast to the first embodiment, a different type of flow restrictor 14 is used. The flow restrictor 14 is formed by a constriction of the main flow path. The flow channel narrows towards the constriction and widens again in a continuous manner to minimize turbulence and deposition of particulate matter onto the walls of the flow channel. In the present example, the flow sensor 15 is arranged in the region of the constriction at the position of minimal cross section. However, the flow sensor may also be placed at another position in the primary flow path 10.

In order to achieve rapid thermalization of the fluid in the secondary flow path, the monitoring device of Fig. 4 comprises a heat exchanger 26. The heat exchanger 26 minimizes temperature gradients along the secondary flow path in a region where the fluid property sensor 24, 25 are arranged. In particular, if the fluid composition sensor 24 is a microthermal sensor, temperature gradients across the sensor should be avoided, since temperature gradients can cause inaccurate outputs of the microthermal sensor. In addition, the heat exchanger may facilitate rapid heat exchange with the environment. The heat exchanger may be formed of a material having high thermal conductivity along the secondary flow path and/or between the secondary flow path and the environment. It may be formed by a portion of the housing that delimits the secondary flow path 20, e.g., by a carrier 60, more specifically, by a printed circuit board, as will be explained in more detail below.

In addition to or as an alternative to the heat exchanger, the monitoring device may comprise a heater 27 for heating the fluid in the secondary flow path. The heater may be operated to avoid condensation of water or some other constituent in the fluid that condensates under the given conditions in the secondary flow path or to remove condensate that may already have formed. The heater 27 is preferably in thermal contact with the heat exchanger 26 to facilitate uniform heating of the fluid in the secondary flow path.

The provision of a heat exchanger and/or heater is advantageous independent of the manner of how the flow restrictor is configured.

## Design of secondary flow path

Figures 5 to 7 illustrate, in highly schematic cross-sectional views, various possible designs of the secondary flow path.

In Figure 5, the secondary flow path 20 is delimited by a housing portion defining a cross-section of the secondary flow path 20 that has a large aspect ratio w/h between the width w and the height $h$ of the secondary flow path 20. A large aspect ratio leads to a large surface area for given cross-sectional area, which helps with efficient thermalization.

Also illustrated is a flow-metering flow path 16 that bypasses the flow restrictor 14 in the main flow path 10.

The flow-metering flow path 16 and the secondary flow path 20 are arranged in such a manner that the flow sensor 15 and the pressure sensor 25 can be mounted on a common carrier 60, e.g., a common printed circuit board. The carrier 60 may act as a heat exchanger for creating a uniform temperature along the secondary flow path.

In Fig. 6, the secondary flow path 20 is formed by slit-like protrusion of the main flow path 10. The flow-metering flow path 16 is formed by a short tube inside the main flow path 10, the tube bypassing the flow restrictor 14.

In Fig. 7, the secondary flow path 20 is formed within the perimeter of a tube that delimits the main flow path 10 and is delimited from the main flow path 10 only by a separating wall within the tube. In this and other embodiments, it is conceivable that the secondary flow path 20 extends over the entire length of the main flow path 10, i.e. the secondary path inlet 21 may be formed at the main path inlet 11, and the secondary path outlet 22 may be formed at the main path outlet 12.

Many other designs for the main flow path and for the secondary flow path are possible. For example, the main flow path may have a polygonal cross section.

Exemplary embodiment

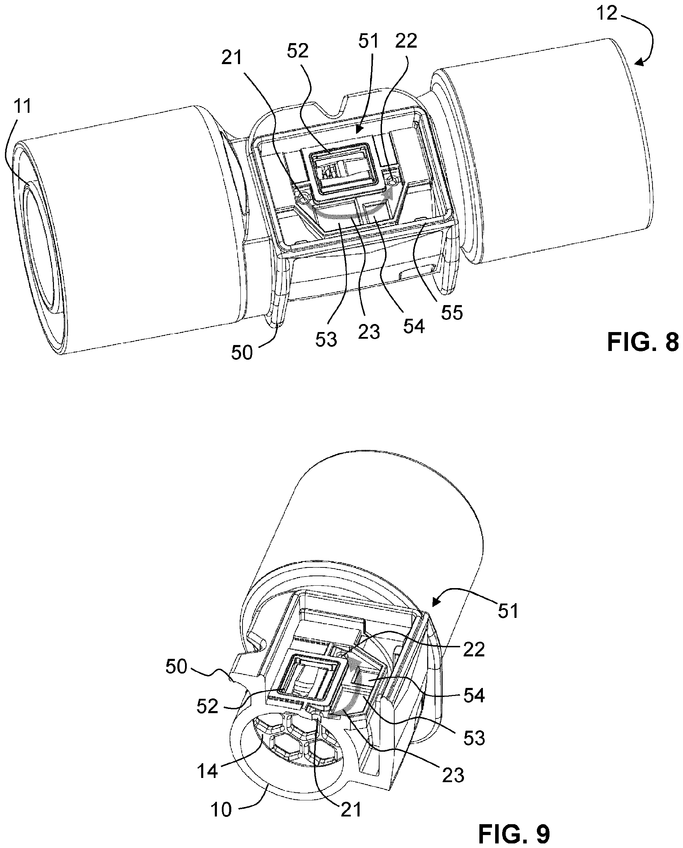

Figures 8 to 11 illustrate a further exemplary embodiment of a monitoring device.

The monitoring device comprises a housing 50. The housing 50 has a tubular portion that defines a main flow path 10, a main path inlet 11 and a main path outlet 12. The main path inlet 11 and the main path outlet 12 are each shaped as a connector for connection to an attachment such as a source of a breathing gas and to a patient interface, respectively. To this end, they are provided with external and internal tapers each to enable quick and secure connections to the attachments. The main path inlet 11 and the main path outlet 12 may furthermore each be surrounded by a cylindrical sleeve, which may be provided with an internal thread for securing the attachments to the monitoring device.

A flow restrictor 14 is inserted into a central part of the tubular portion. Radially outside the central part of the tubular portion, the housing forms a platform-like receptacle 51. The receptacle 51 is formed in one piece with the tubular portion. It is delimited by a circumferential rim 55.

A first cavity 52 is formed in the receptacle 51. The first cavity 52 has an opening towards the main flow path 10. The first cavity 52 is shaped and sized to receive a flow sensor 15 in a fluid-tight manner, such that the flow sensor 15 is exposed to the fluid flow in the main flow path while fluid from the main flow path 10 cannot enter the receptacle 51 through the first cavity 52.

The receptacle further defines two second flat cavities 53, 54. These cavities are not open towards the main flow path 10. They are shaped and sized to receive a fluid composition sensor 24 and a pressure sensor 25 in such a manner that fluid in the secondary flow path 20 can get into contact with these sensors.

Two openings connect the inside of the tubular portion with the inside of the receptacle 51. One of these openings is located upstream of the flow restrictor 14 with respect to the main path and forms the secondary path inlet 21. The other opening is located downstream of the flow restrictor 14 and forms the secondary path outlet 22. The secondary flow path 20 thus extends through the inside of the receptacle 51.

As illustrated in Fig. 10, the flow sensor 15, the fluid composition sensor 24 and the pressure sensor 25 are mounted on a common carrier 60 in the form of a printed circuit board (PCB). The carrier 60 is shaped and sized to be received in the receptable 51.

When the carrier 60 is received in the receptable, the flow sensor 15 is received in the first cavity in a fluid-tight manner. The fluid composition sensor 24 and the pressure sensor 25 are received in the second cavities at a small distance from the bottom of each of these cavities. This distance can readily be adjusted to adjust the flow resistance of the secondary flow channel.

The carrier 60 may comprise at least one metal layer 61 that is patterned to form a continuous, uninterrupted heat conduction path between a portion of the secondary flow path upstream of the sensors 24, 25 and a portion downstream of these sensors to facilitate rapid heat exchange between the upstream and downstream portions.

As illustrated in Fig. 11, the carrier 60 may further have one or more through-holes 62 that are filled with a material having high thermal conductivity, in particular, a metal, to facilitate heat exchange between the opposite sides of the carrier 60. The metal may extend in a plane parallel to the plane of the carrier on each side of the carrier, forming a contact portion 63 extending in a plane parallel to the plane of the carrier for increasing the thermal contact with the fluid in the secondary flow path and with the environment.

The carrier 60 may be part of a lid forming a connector, as it is known per se from DE202014103998A1.

Thermal flow sensor

As already discussed above, the flow sensor 15 may be a microthermal sensor. Likewise, the fluid composition sensor 24 may be a microthermal sensor. Also the pressure sensor 25 may employ a microthermal sensor.

Fig. 12 illustrates an example of a microthermal sensor 70 that can be used as the flow sensor 15, as a fluid composition sensor 24 or as part of the pressure sensor 25. The microthermal sensor comprises a heater bridge 71 with a heater 72, sandwiched between two measurement bridges 73 and 75. The heater bridge 71 and the measurement bridges 73, 75 each span an opening or recess 78 in a substrate 77. The substrate may be a semiconductor chip, in particular, a silicon chip. The ends of the bridges are anchored in or on the substrate 77, whereby the bridges are suspended over the opening or recess 78. The bridges are mutually separated by voids (gaps). The bridges are arranged in a common plane. Each bridge may be formed by a plurality of dielectric layers, metal layers and/or polysilicon layers patterned from a layer stack on the substrate 77. The stack may be a CMOS layer stack. The layer stack may have been applied to the semiconductor chip and processed by typical CMOS methodology, as it is well known in the art. Further details about the setup and fabrication of a sensor device comprising several bridges than span an opening or recess are disclosed in EP3367087A2, whose contents are incorporated herein by reference in their entirety.

Each of the two measurement bridges 73, 75 carries a temperature sensing element 74, 75, respectively, in the form of a pair of thermopiles. The hot junctions of each thermopile are arranged near the center of each measurement bridge. The cold junctions of all thermopiles are arranged on the substrate 77, which effectively acts as a heat sink with very large heat capacity and may therefore be assumed to have the same temperature $T_a$ for all thermopiles.

In operation, the heater element 72 is supplied with heating power to heat the heater bridge 71. The heating power may be provided, e.g., with a predetermined current, or the heating power may be regulated such that the heater element 72 assumes a predetermined temperature. For determining and regulating the temperature of the heater bridge 71, the resistance of the heater element 72 may be determined, the resistance being a measure of said temperature. When the heater element 72 is heated, heat flow occurs from the heater bridge 71 through the fluid sample into each of the measurement bridges 73, 75. After transient effects have decayed, the hot junctions of the temperature sensing elements 74, 76 on each measurement bridge 73, 75 assume a certain temperature $T_1$, $T_2$.

The resulting thermoelectric voltages of the temperature sensing elements 74, 76 are determined. These thermoelectric voltages depend on the thermal conductivity of the fluid and can therefore be used to determine the thermal conductivity of the fluid. Thermal conductivity in turn depends on the composition of the fluid. A measurement of thermal conductivity therefore allows conclusions about the composition of the fluid. The difference of the thermoelectric voltages depends on the mass flow rate along flow direction F. By calibration measurements using fluids with known thermal conductivities and known mass flow rates, correlations between the values of the thermal signals, thermal conductivity and mass flow rate can readily be established.

Processing circuitry

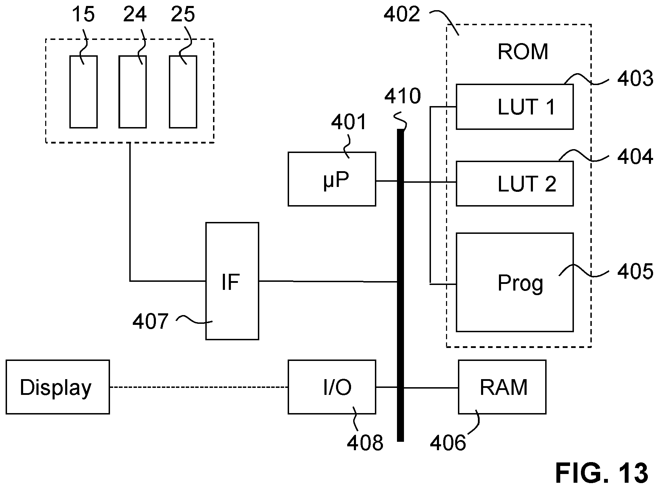

Figure 13 illustrates, in a highly schematic manner, a block diagram of a possible embodiment of the processing circuitry 40. The processing circuitry comprises a processor ($\mu$P) 401, a non-volatile (e.g., Flash ROM) memory 402, and a volatile (RAM) memory 406. The processor $\mu$P communicates with the memory devices 402, 406 via a bus 410. The non-volatile memory 402 stores, inter alia, plural lookup tables (LUT), only two such lookup tables 403, 404 being illustrated. The lookup tables may store, for example, calibration data relating the thermal signal to actual values of the mass flow rate and/or the thermal conductivity and/or to another target value that correlates with thermal conductivity, such as the concentration of a selected target gas. The non-volatile memory 402 further stores a machine-executable program (Prog) 405 for execution in the processor $\mu$P. Via a device interface (IF) 407, the processing circuitry operates the flow sensor 15, the concentration sensor 24 and the pressure sensor 25. A wired or wireless input/output interface I/O 408 enables communication to the outside world, e.g., with a display device 420.

The processing circuitry 40 may comprise an integrated circuit that is disposed on the PCB 60 together with the flow sensor 15, the concentration sensor 24, and the pressure sensor 25. In other embodiments, the processing circuitry 40 may be integrated on a common silicon chip together with the flow sensor 15, the concentration sensor 24, or the pressure sensor 25. In yet other embodiments, at least parts of the processing circuitry 40 may be implemented separately, e.g., by a separate general-purpose computer.

The processing circuitry may be operable to discriminate between inhalation and exhalation events based on the measured flow rate profile and to determine separate output data for inhalation events and exhalation events. For example, the processing circuitry may be operable to determine one or more of the following parameters during inhalation events: peak or mean inhalation flow, peak or mean pressure during inhalation, molecular oxygen content, argon content, humidity. Furthermore, the processing circuitry may be operable to determine one or more of the following parameters during exhalation events: peak or mean exhalation flow, peak or mean pressure during exhalation, carbon dioxide content, nitric oxide content, acetone content, humidity. The processing circuitry may further be operable to determine the period between subsequent inhalation or exhalation events from the flow rate profile. The processing circuitry may be operable to output these parameters and/or any other parameters that have been derived from these parameters.

Modifications

From the above description it is apparent that the invention is not limited to the above-described exemplary embodiments, and that many modifications are possible.

In particular, the use of the monitoring device is not restricted to resuscitation systems. The monitoring device may be useful in a variety of other applications, including but not limited to other medical applications like mechanical ventilation of patients in hospitals.

**Claims**

1. A monitoring device for monitoring a flow of a fluid, in particular, a breathing gas, the device comprising:

   a main flow path (10) for the fluid between a main path inlet (11) and a main path outlet (12);
   a flow sensor (15) coupled to the main flow path (10) for measuring a fluid flow rate (13) in the main flow path (10);
   a flow restrictor (14) arranged in the main flow path (10) to cause a pressure difference across the flow restrictor (14) when the fluid flows along the main flow path (10);
   a secondary flow path (20) for the fluid, the secondary flow path being in fluidic communication with the main flow path (10), the secondary flow path (20) bypassing the flow restrictor (14), the secondary flow path (20) having higher flow resistance than the main flow path (10); and
   at least one fluid property sensor (24; 25) coupled to the secondary flow path (20) for determining at least one property of the fluid in the secondary flow path (20).

2. The monitoring device of claim 1,

   wherein the main flow path (10) includes a flow-metering flow path (16) that bypasses the flow restrictor (14), and
   wherein the flow sensor (15) is arranged in the flow-metering flow path (16),
   wherein preferably the flow-metering flow path (16) branches off from the main flow path (10) downstream of a position where the secondary flow path (20) branches off from the main flow path (10) and opens out into the main flow path (10) upstream of a position where the secondary flow path (20) opens out into the main flow path, wherein preferably the secondary flow path (20) has higher flow resistance than the flow-metering flow path (16).

3. The monitoring device of claim 1 or 2, comprising a heat exchanger (26) arranged to be in thermal contact with the fluid in the secondary flow path (20), the heat exchanger (26) being configured to reduce temperature gradients along the secondary flow path (20) in a region where the at least one fluid property sensor (24; 25) is arranged and/or to facilitate heat exchange between the secondary flow path (20) and an environment outside of the monitoring device,

   wherein the heat exchanger (26) preferably comprises a material having a thermal conductivity of at least 10 W/(m · K) extending along at least a portion of the secondary flow path (20) between an upstream portion of the secondary flow path upstream of the at least one fluid property sensor (24; 25) and a downstream portion of the secondary flow path (20) downstream of the at least one fluid property sensor (24; 25) and/or extending between the secondary flow path (20) and the environment,
   wherein the heat exchanger (16) preferably has a heat transfer coefficient between the upstream and downstream portions of the secondary flow channel (20) that is higher than a heat transfer coefficient due to forced convection of the fluid through the secondary flow channel (20),
   wherein the heat exchanger (16) preferably comprises a carrier (60) on which the flow sensor (15) and/or the at least one fluid property sensor (24; 25) are mounted, in particular, a circuit board comprising at least one metal layer (61) that defines an uninterrupted heat conduction path between the upstream and downstream portions of the secondary flow channel (20).

4. The monitoring device of claim 3, wherein the secondary flow path (20) has a cross section having an aspect ratio of at least 5:1 in a region where the secondary flow path (20) is in contact with the heat exchanger (26).

5. The monitoring device of any one of the preceding claims, comprising a heater (27) for heating the fluid in the secondary flow path,

   wherein the heater (27) preferably is in thermal contact with the heat exchanger (26) if the monitoring device is configured according to claim 3 or 4,
   wherein the heater preferably is mounted on the carrier (60) if the heat exchanger comprises a carrier (60) on which the flow sensor (15) and/or the at least one fluid property sensor (24; 25) are mounted.

6. The monitoring device of any one of the preceding claims, wherein the at least one fluid property sensor (24; 25) includes a pressure sensor (25) for measuring a fluid pressure in the secondary flow path.

7. The monitoring device of claim 6, wherein the pressure sensor (25) is arranged to measure the fluid pressure ($p_{sensor}$) in the secondary flow path (20) at a position that corresponds to a fraction $\kappa_{opt} = -(1 + \zeta_B)/\zeta_A$ of a total length of the secondary flow path (20), measured from a downstream end of the secondary flow path (20), wherein $\zeta_A$ is a pressure loss coefficient at the flow restrictor (14) and $\zeta_B$ is a pressure loss coefficient at the main path outlet (12).

8. The monitoring device of any one of the preceding claims,

wherein the at least one fluid property sensor (24; 25) includes a fluid composition sensor (24) for determining a composition of the fluid in the secondary flow path (20),
in particular, wherein the fluid composition sensor (24) is configured to determine a concentration of at least one of the following components of the fluid in the secondary flow path (20):

carbon dioxide;
molecular oxygen;
argon;
nitric oxide;
acetone; and
humidity.

9. The monitoring device of claim 8, wherein the fluid composition sensor (24) is a thermal sensor.

10. The monitoring device of any one of the preceding claims, wherein the monitoring device is configured to carry out the steps of:

for a plurality of time intervals, operating the flow sensor and the at least one fluid property sensor to obtain the fluid flow rate in the main flow path and the at least one property of the fluid in the secondary flow path;
determining a flow direction in the primary flow channel based on signals from the flow sensor to discriminate between inhalation and exhalation events;
determining separate output data for the inhalation and exhalation events of one or more of the breathing cycles based on the fluid flow rate and/or the at least one property of the fluid; and
optionally, outputting the output data.

11. The monitoring device of any one of the preceding claims, comprising a carrier (60) on which the flow sensor (15) and the at least one fluid property sensor (24; 25) are mounted,

wherein the monitoring device comprises a housing (50), the housing defining the main flow path (10), the housing (50) further comprising a receptacle (51) for receiving the carrier (60),
wherein openings between the receptacle (51) and the main flow path (10) are formed in the housing (50), the openings forming a secondary path inlet (21) and a secondary path outlet (22) such that the secondary flow path (20) passes through the receptacle (51),
wherein the receptacle (51) defines a first cavity (52) for receiving the flow sensor (15), the cavity (52) having an opening towards the primary flow path (10),
wherein the flow sensor (15) is preferably received in the first cavity (52) in a fluid-tight manner when the carrier (60) with the flow sensor (15) and the at least one fluid property sensor (24; 25) mounted thereon is received in the receptacle (51),
wherein the at least one fluid property sensor (24; 25) is in contact with the secondary flow path (20) when the carrier (60) with the flow sensor (15) and the at least one fluid property sensor (24; 25) mounted thereon is received in the receptacle (51), and
wherein the receptacle optionally defines at least one second cavity (53; 54) for receiving the at least one fluid property sensor (24; 25), and wherein preferably the at least one fluid property sensor (24; 25) is arranged in the at least one second cavity (53; 54) in such a manner that the at least one fluid property sensor (24; 25) is able to interact with the fluid in the secondary flow path (20) when the carrier (60) with the flow sensor (15) and the at least one fluid property sensor (24; 25) mounted thereon is received in the receptacle (51).

12. The monitoring device of any one of the preceding claims,

wherein the main path inlet (11) is shaped as a connector for connection to a source of a breathing gas, and
wherein the main path outlet (12) is shaped as a connector for attachment to a patient interface (30).

13. A ventilation system for ventilating a patient, in particular, a mobile ventilation system for resuscitation, comprising:

the monitoring device of any one of the preceding claims;
a patient interface (30) attached to the main path outlet (12), in particular, a breathing mask, and optionally, a source of a breathing gas, in particular, manual breathing gas delivery device comprising a self-inflating bag.

14. The ventilation system of claim 13,

wherein the at least one fluid property sensor (24; 25) includes a pressure sensor (25) for measuring a fluid pressure ($p_{sensor}$) in the secondary flow path (20), and
wherein the pressure sensor (25) is arranged to measure the fluid pressure ($p_{sensor}$) in the secondary flow path at a position where said fluid pressure ($p_{sensor}$) substantially corresponds to a pressure ($p_{mask}$) of the fluid in the patient interface (30) when a patient receives the fluid through the monitoring device and the patient interface (30).

15. A method of operating a ventilation system according to claim 13 or 14, the method comprising carrying out the following steps during a plurality of breathing cycles, each breathing cycle comprising an inhalation event and an exhalation event:

operating the flow sensor (15) and the at least one fluid property sensor (24; 25) to obtain the fluid flow rate in the main flow path (10) and the at least one property of the fluid in the secondary flow path (20) during inhalation events and exhalation events;
determining a flow direction in the primary flow channel based on signals from the flow sensor (15) to discriminate between inhalation and exhalation events;
determining separate output data for the inhalation and exhalation events of one or more of the breathing cycles based on the fluid flow rate and the at least one property of the fluid; and
optionally, outputting the output data.

FIG. 1

FIG. 2

FIG. 3

**FIG. 4**

**FIG. 5**

**FIG. 6**

**FIG. 7**

FIG. 8

FIG. 9

FIG. 10

FIG. 11

FIG. 12

FIG. 13

Europäisches
Patentamt

European
Patent Office

Office européen
des brevets

## EUROPEAN SEARCH REPORT

Application Number

EP 23 18 6128

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 7 520 051 B2 (HONEYWELL INT INC [US])<br>21 April 2009 (2009-04-21)<br>* column 1, lines 20-26; figures 1-7 *<br>* column 3, line 1 - column 4, line 6 *<br>----- | 1,2,6,8,<br>9,11-13<br>7,10,14,<br>15 | INV.<br>A61M16/08<br>A61M16/00<br>G01F1/36<br>G01F1/40 |
| X<br>A | US 2020/046256 A1 (ALTOBELLI DAVID E [US]<br>ET AL) 13 February 2020 (2020-02-13)<br>* paragraphs [0047] - [0049], [0052] -<br>[0055], [0072], [0085] - [0097],<br>[0100], [0112], [0113]; figures 16-19,26<br>*<br>----- | 1-5,8,<br>12,13<br>7,10,14,<br>15 | G01F1/42<br>G01F1/44<br>G01F1/684<br>G01F1/688<br>G01F5/00<br>G01F15/00<br>G01F15/14 |
| A | US 2018/180455 A1 (NAKAO HIDEYUKI [JP] ET<br>AL) 28 June 2018 (2018-06-28)<br>* paragraphs [0054] - [0057], [0067],<br>[0105], [0106], [0108] - [0115],<br>[0135]; figures 4-12,19,20,22 *<br>----- | 1-15 | |
| A | US 2011/257898 A1 (OOISHI YASUHARU [JP])<br>20 October 2011 (2011-10-20)<br>* paragraphs [0278], [0279]; figures 1-47<br>*<br>----- | 1-15 | |
| A | US 2016/245681 A1 (MAGINNIS THOMAS O [US]<br>ET AL) 25 August 2016 (2016-08-25)<br>* paragraphs [0002], [0003], [0004],<br>[0029], [0057]; figures 1,7 *<br>----- | 1-15 | TECHNICAL FIELDS<br>SEARCHED (IPC)<br><br>A61M<br>G01F |
| A | US 2011/125424 A1 (HAN HUAICHENG [CN] ET<br>AL) 26 May 2011 (2011-05-26)<br>* paragraph [0019]; figures 1,2 *<br>----- | 1-15 | |
| A | US 9 091 577 B2 (HONEYWELL INT INC [US])<br>28 July 2015 (2015-07-28)<br>* column 4, line 29 - column 9, line 45;<br>figures 3-7 *<br>-----<br>-/-- | 1,6,7,<br>13,14 | |

The present search report has been drawn up for all claims

| Place of search | Date of completion of the search | Examiner |
|---|---|---|
| The Hague | 19 March 2024 | Rambaud, Dilek |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

& : member of the same patent family, corresponding
     document

EPO FORM 1503 03.82 (P04C01)

**page 1 of 2**

## EUROPEAN SEARCH REPORT

Application Number

EP 23 18 6128

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 6 722 359 B2 (SAIME SARL [FR]) 20 April 2004 (2004-04-20) * figures 1,2,14,15 * | 1-15 | |
| A | US 2019/254534 A1 (KOLTOWSKI LUKASZ [PL] ET AL) 22 August 2019 (2019-08-22) * paragraphs [0060], [0065], [0066], [0069], [0073], [0074], [0076] - [0078], [0093], [0094], [0111]; figures 1,2,4 * | 1-15 | |
| A | US 2022/062571 A1 (NATARAJ CHANDRASEKHAR [US] ET AL) 3 March 2022 (2022-03-03) * paragraphs [0035], [0036], [0046], [0050], [0051], [0058], [0059]; figures 2,5,8 * | 1-15 | |
| A | AU 2015 201 331 A1 (RESMED LTD) 2 April 2015 (2015-04-02) * paragraphs [0158], [0169] - [0172]; figures 46,52-58 * | 1-15 | TECHNICAL FIELDS SEARCHED (IPC) |
| A | US 2014/238398 A1 (CHRISTOPHER KENT L [US] ET AL) 28 August 2014 (2014-08-28) * paragraphs [0070] - [0094]; figure 3 * | 1-15 | |
| A | WO 2023/049958 A1 (RESMED PTY LTD [AU]) 6 April 2023 (2023-04-06) * paragraph [0224]; figures 4A-4E * | 1-15 | |

The present search report has been drawn up for all claims

| Place of search | Date of completion of the search | Examiner |
|---|---|---|
| The Hague | 19 March 2024 | Rambaud, Dilek |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

**Application Number**

EP 23 18 6128

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

**LACK OF UNITY OF INVENTION
SHEET B**

Europäisches
Patentamt
European
Patent Office
Office européen
des brevets

The Search Division considers that the present European patent application does not comply with the requirements of unity of invention and relates to several inventions or groups of inventions, namely:

```
1. claims: 2-5, 8, 9, 11, 12(completely); 1, 6, 13(partially)

   A monitoring device comprising a main channel, a flow
   restrictor in the main channel, a secondary channel, wherein
   a flow sensor is coupled to the main channel and at least a
   fluid property sensor coupled to the secondary channel,
   wherein the secondary channel comprises a heat exchanger in
   the secondary channel.
                         ---


2. claims: 7, 14(completely); 1, 6, 13(partially)

   A monitoring device comprising a main channel, a flow
   restrictor in the main channel, a secondary channel, wherein
   a flow sensor is coupled to the main channel and at least a
   fluid property sensor coupled to the secondary channel with
   pressure measurements in the secondary channel to represent
   the static pressure inside the patient interface
                         ---


3. claims: 10, 15(completely); 1, 13(partially)

   A monitoring device comprising a main channel, a flow
   restrictor in the main channel, a secondary channel, wherein
   a flow sensor is coupled to the main channel and at least a
   fluid property sensor coupled to the secondary channel
   configured to determine the flow direction, inhalation,
   exhalation events
                         ---
```

## ANNEX TO THE EUROPEAN SEARCH REPORT
## ON EUROPEAN PATENT APPLICATION NO.

EP 23 18 6128

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2024

| Patent document cited in search report | | Publication date | Patent family member(s) | | | Publication date |
|---|---|---|---|---|---|---|
| US 7520051 | B2 | 21-04-2009 | CN | 101216331 | A | 09-07-2008 |
| | | | EP | 2100104 | A2 | 16-09-2009 |
| | | | US | 2008163683 | A1 | 10-07-2008 |
| | | | WO | 2008086045 | A2 | 17-07-2008 |
| US 2020046256 | A1 | 13-02-2020 | CA | 2641608 | A1 | 16-08-2007 |
| | | | EP | 1986549 | A2 | 05-11-2008 |
| | | | EP | 2404552 | A1 | 11-01-2012 |
| | | | JP | 2009525774 | A | 16-07-2009 |
| | | | US | 2007185405 | A1 | 09-08-2007 |
| | | | US | 2013331724 | A1 | 12-12-2013 |
| | | | US | 2015080676 | A1 | 19-03-2015 |
| | | | US | 2020046256 | A1 | 13-02-2020 |
| | | | WO | 2007092052 | A2 | 16-08-2007 |
| US 2018180455 | A1 | 28-06-2018 | EP | 3321646 | A1 | 16-05-2018 |
| | | | JP | 6493235 | B2 | 03-04-2019 |
| | | | JP | 2017129470 | A | 27-07-2017 |
| | | | US | 2018180455 | A1 | 28-06-2018 |
| | | | WO | 2017126269 | A1 | 27-07-2017 |
| US 2011257898 | A1 | 20-10-2011 | CN | 102253080 | A | 23-11-2011 |
| | | | EP | 2381248 | A1 | 26-10-2011 |
| | | | JP | 5534193 | B2 | 25-06-2014 |
| | | | JP | 2011226927 | A | 10-11-2011 |
| | | | US | 2011257898 | A1 | 20-10-2011 |
| US 2016245681 | A1 | 25-08-2016 | CN | 107407590 | A | 28-11-2017 |
| | | | DE | 112016000872 | T5 | 16-11-2017 |
| | | | JP | 6731936 | B2 | 29-07-2020 |
| | | | JP | 2018506042 | A | 01-03-2018 |
| | | | TW | 201643382 | A | 16-12-2016 |
| | | | US | 2016245681 | A1 | 25-08-2016 |
| | | | WO | 2016137826 | A1 | 01-09-2016 |
| US 2011125424 | A1 | 26-05-2011 | CN | 101354273 | A | 28-01-2009 |
| | | | EP | 2312276 | A1 | 20-04-2011 |
| | | | JP | 5425902 | B2 | 26-02-2014 |
| | | | JP | 2011528104 | A | 10-11-2011 |
| | | | US | 2011125424 | A1 | 26-05-2011 |
| | | | WO | 2010006474 | A1 | 21-01-2010 |
| US 9091577 | B2 | 28-07-2015 | AU | 2012200528 | A1 | 16-08-2012 |
| | | | CN | 102645249 | A | 22-08-2012 |
| | | | CN | 106840296 | A | 13-06-2017 |
| | | | EP | 2482043 | A1 | 01-08-2012 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 23 18 6128

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2024

| Patent document cited in search report | | | Publication date | Patent family member(s) | | | Publication date |
|---|---|---|---|---|---|---|---|
| | | | | EP | 3136062 | A1 | 01-03-2017 |
| | | | | US | 2012192642 | A1 | 02-08-2012 |
| | | | | US | 2013213131 | A1 | 22-08-2013 |
| US | 6722359 | B2 | 20-04-2004 | DE | 60102422 | T2 | 04-05-2005 |
| | | | | EP | 1177810 | A1 | 06-02-2002 |
| | | | | ES | 2215865 | T3 | 16-10-2004 |
| | | | | FR | 2812203 | A1 | 01-02-2002 |
| | | | | JP | 4689893 | B2 | 25-05-2011 |
| | | | | JP | 2002119595 | A | 23-04-2002 |
| | | | | US | 2002014239 | A1 | 07-02-2002 |
| US | 2019254534 | A1 | 22-08-2019 | AU | 2017347595 | A1 | 18-04-2019 |
| | | | | BR | 112019007685 | A2 | 02-07-2019 |
| | | | | CA | 3038009 | A1 | 26-04-2018 |
| | | | | CN | 109937000 | A | 25-06-2019 |
| | | | | EP | 3528697 | A2 | 28-08-2019 |
| | | | | ES | 2961744 | T3 | 13-03-2024 |
| | | | | IL | 265939 | A | 30-05-2019 |
| | | | | JP | 2020500049 | A | 09-01-2020 |
| | | | | KR | 20190065316 | A | 11-06-2019 |
| | | | | RU | 2019108767 | A | 20-11-2020 |
| | | | | US | 2019254534 | A1 | 22-08-2019 |
| | | | | US | 2023213368 | A1 | 06-07-2023 |
| | | | | WO | 2018073343 | A2 | 26-04-2018 |
| US | 2022062571 | A1 | 03-03-2022 | US | 2022062571 | A1 | 03-03-2022 |
| | | | | WO | 2022046983 | A1 | 03-03-2022 |
| AU | 2015201331 | A1 | 02-04-2015 | NONE | | | |
| US | 2014238398 | A1 | 28-08-2014 | US | 2014238398 | A1 | 28-08-2014 |
| | | | | US | 2017128692 | A1 | 11-05-2017 |
| WO | 2023049958 | A1 | 06-04-2023 | NONE | | | |

EPO FORM P0459

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020118138 A1
- WO 2014078840 A1
- DE 202014103998 A1
- EP 3367087 A2